# FASCICULE DE BREVET EUROPEEN

(11) **EP 0 780 223 B1**
(45) Date de publication et mention de la délivrance du brevet: **27.10.2004**
(21) Numéro de dépôt: 96402819.5
(22) Date de dépôt: 19.12.1996
(51) Int. Cl.: B32B 9/04, B32B 25/04, A61F 6/04, A61L 31/00, A61B 19/04

(54) **Matériau multicouche, procédé de préparation et applications**
Mehrschichtwerkstoff, Verfahren zur Herstellung, und Anwendungen
Multilayered material, method for producing and applications

(30) Priorité: 20.12.1995 FR 9515166
(43) Date de publication de la demande: 25.06.1997
(73) Titulaire: HUTCHINSON, 75008 Paris (FR)
(72) Inventeur: Hoerner, Pierre, 68180 Horbourg-Wihr (FR); Riess, Gérard, 68200 Mulhouse (FR); Busnel, René Guy, 91570 Bievres (FR); Cheymol, André, 86220 Dange Saint Romain (FR)
(74) Mandataire: Orès, Bernard

(56) Documents cités:
- EP-A- 0 443 870
- EP-A- 0 604 103
- WO-A-91/15362
- US-A- 5 357 636

## Description

La présente invention est relative à des films d'élastomère multicouche (matériau multicouche) comprenant au moins une couche continue chargée en substance chimique active (comme des anti-corrosifs, des lubrifiants ou encore des biocides à usage médical), insérée entre des couches barrières d'élastomère inertes ; elle couvre également les procédés de préparation ainsi que les diverses applications de ces films.

Les différents matériaux élastomères, habituellement utilisés dans le domaine médical ou paramédical peuvent être modifiés, de manière à être associés à des substances chimiques actives ayant un effet de protection, lors de l'utilisation de ces matériaux (gants, doigtiers, préservatifs...), comme illustré dans le Brevet Européen 306 389 ou la Demande Internationale WO 95/17453, au nom de la Demanderesse, qui décrivent des films d'élastomère incluant une dispersion d'au moins une substance chimique active, laquelle dispersion étant soit sous forme liquide (Demande Internationale WO 95/17453), soit contenue dans des microcapsules, dont les parois se rompent sous l'action de forces de frottement ou de cisaillement (Brevet européen 306 389).

Toutefois, la réalisation de films d'élastomère selon les méthodes décrites dans les demandes et brevets précités présente un certain nombre d'inconvénients, qui concernent soit le produit lui-même, c'est à dire le film, soit le procédé d'obtention du film.
* inconvénients liés au produit :
   - l'inconvénient majeur des films selon l'une quelconque des deux méthodes précitées est l'inhomogénéité de la répartition des substances chimiques actives, au sein du matériau polymérique, due à ce que la substance chimique est dispersée dans le matériau polymérique, sous forme de gouttelettes ou de microcapsules.

   Cette non-uniformité de la répartition des substances chimiques actives se traduit par des lacunes en substance chimique active, qui constituent un risque dans le cadre de la protection recherchée, même si ce risque est statistiquement très faible ;
   - les microcapsules contenues dans les films d'élastomère selon le Brevet Européen 306 389 peuvent présenter des difficultés à se rompre, pour libérer leurs substances actives, lors de la perforation desdits films ;
   - les films d'élastomère contenant des inclusions liquides selon la Demande Internationale WO 95/17453 mettent en oeuvre des stabilisants macromoléculaires de type copolymère greffé ou séquencé (copolymère bloc) qui sont d'un coût relativement élevé.
* inconvénients liés au procédé :
   - dans le cas de la Demande internationale WO 95/17453, la préparation de films à partir d'une dispersion de substance chimique active, est conditionnée par la stabilité du bain contenant la dispersion, elle-même fonction de la teneur en substance active (virucide, par exemple), ce qui constitue un facteur limitant ;
   - de même, dans le Brevet 306 389, l'obtention de microcapsules, de taille controlée nécessite la mise en oeuvre d'un processus complexe, particulièrement au niveau du contrôle de l'épaisseur et de la porosité de la paroi ;
   - la réalisation de films selon l'une quelconque des deux méthodes s'accompagne dans tous les cas d'émission de vapeurs organiques ; en effet, les élastomères sont toujours utilisés sous forme de solution plus ou moins concentrée dans des solvants organiques.

En conséquence, poursuivant ses recherches, la Demanderesse s'est donné pour but la mise au point d'un nouveau type de matériau élastomère répondant mieux aux besoins de la pratique que l'ensemble des élastomères de l'Art antérieur, notamment en ce qu'il inclut au moins une substance chimique active, dans les proportions désirées, sous la forme d'une couche continue et non d'une dispersion ou d'une émulsion, ce qui permet d'éviter les problèmes liés à l'étape de dispersion et/ou d'émulsification (stabilité du bain d'émulsion, coût des polymères stabilisants, présence de lacunes) et en ce qu'il permet d'obtenir un matériau multicouches, souple et élastique, particulièrement bien adapté à la préparation de gants, doigtiers ou préservatifs, à partir desquels, la substance active x n'est libérée qu'en cas de rupture du matériau.

De plus, le procédé de préparation du matériau selon la présente invention peut être effectué entièrement ou de façon partielle en milieu aqueux.

La présente invention a pour objet un film polymérique multicouche (matériau multicouche), caractérisé en ce qu'il comprend au moins une couche A constituée d'un gel non-réversible en fonction de la température, et chargé en au moins une substance chimique active x et au moins deux couches barrière E, comprenant un élastomère de synthèse e, la solidarisation desdites couches entre elles étant assurée, par un agent de collage polymérique y, incorporé dans au moins l'une desdites couches A ou E et/ou par une couche de collage Z indépendante, comprenant un polymère z, et/ou par un traitement chimique ou physique d'au moins l'une desdites couches A ou E.

On entend par traitement chimique soit un greffage, soit une attaque chimique à l'aide d'un acide, par exemple l'acide sulfurique, et par traitement physique, un bombardement de la surface du film avec des ions ou des électrons : traitement Corona ou Plasma ou des photons : traitement ultraviolet.

Les gels non-réversibles, en fonction de la température, sont formés par voie chimique, c'est-à-dire par réticulation par liaison covalente, qui se produit par ajout d'un réticulant ri (réticulant irréversible) et/ou par activation thermique ou photochimique, comme par exemple sous l'effet d'un rayonnement ultra-violet.

Selon un mode de réalisation avantageux de ladite couche A, le gel non-réversible comprend essentiellement un polymère structurant c, un réticulant ri, au moins une substance chimique active x et un solvant sₐ ; il peut contenir, en outre, un agent de collage y. L'ensemble forme alors une couche A souple et élastique.

Dans certains cas, le polymère structurant c joue également le rôle de solvant sₐ.

Selon une disposition avantageuse de ce mode de réalisation, le polymère structurant c est un polymère non miscible avec l'élastomère e, de préférence de type hydrophile et compatible avec les substances chimiques actives x, seules ou en solution dans un solvant sₐ.

Selon une modalité avantageuse de cette disposition, ledit polymère structurant c est sélectionné dans le groupe constitué par (i) les polymères d'origine naturelle comme la gélatine, les gommes, les pectines, les alginates, les polypeptides, les polyurées, les héparinoïdes et le polyacide gluconique ou certains dérivés cellulosiques comme la méthylcellulose, l'hydroxyéthylcellulose, l'hydroxypropyl-cellulose et la carboxyméthylcellulose ou certains dérivés de l'amidon et (ii) les polymères de synthèse comme le polyéthyleneimine, l'acide polyacrylique, l'alcool polyvinylique, seuls ou en réseaux interpénétrés, les polyacrylamides et leurs dérivés, la polyvinyl pyrrolidone, le polydiméthyl siloxane, les poly(vinyléthers) comme le poly(vinylméthyléther).

Selon une autre disposition avantageuse de ce mode de réalisation, le réticulant ri est sélectionné, pour les polymères c comportant des fonctions hydroxyles, parmi les aldéhydes ou dialdéhydes à faible ou à haut poids moléculaire comme par exemple le glyoxal ou d'autres composés tels les dérivés urée formol ou mélamine formol, et pour les polymères comportant des fonctions hydroxyles, carboxyliques ou amines, parmi des prépolymères comportant des fonctions isocyanates ou parmi les méthacrylates, les imides ou les persulfates et d'une façon générale parmi tout autre composé susceptible de réagir chimiquement avec le polymère structurant c.

Conformément à l'invention, la couche A comprend en outre :
- au moins une substance chimique active x, sélectionnée parmi les composés capables de provoquer une dénaturation quasi-instantanée des protéines par simple contact, soit par réaction chimique ou par effet physicochimique comme par modification de la tension de surface. Cette famille de composés comprend entre autres les biocides ; ladite substance chimique active x doit être adaptée au polymère structurant c et aux autres constituants de la couche A, ceci afin d'éviter toute possibilité de réaction chimique entre-eux.

Selon une disposition avantageuse, ladite substance chimique active est un ammonium quaternaire, de préférence du diméthyldidécylammonium (Bardac®), des biguanides, le phtalaldéhyde, des dérivés phénoliques, le formol, des tensio-actifs non ioniques comportant au moins une séquence polyoxyéthylène, l'hexamidine, des composés iodés, par exemple le complexe iodé de la polyvinylpyrrolidone, des tensio-actifs non ioniques à activité virucide, les bichromates et hypochlorites de sodium et de potassium ou des polymères chargés comme les polyélectrolytes présentant des activités biocides comme le poly(acide acrylique), le polychlorure de triméthylammonium de divinyl benzyle, le copolymère de divinyl éther-anhydride maléique, les polyéthylène imines, les polyéther amines, les polyurées et les polypeptides, utilisés seuls ou en mélange.

Dans certains cas, la substance chimique active x joue également le rôle de solvant sₐ.
- un solvant sₐ, sélectionné dans le groupe constitué par (i) les composés peu volatils comme les polyols, de préférence parmi l'éthylène glycol, le propylène glycol, la glycérine et plus généralement les polyéthylène glycols liquides à température ambiante et de masse moléculaire comprise entre 62 (éthylène glycol) et 750 daltons (PEG750), éventuellement l'eau lorsqu'elle est incluse dans le polymère structurant c et (ii) des solvants volatils tels que les alcools ou les cétones à faible poids moléculaire, les composés éthérés non cycliques ou cycliques comme le tétrahydrofurane, (iii) le diméthylformamide, le diméthyl sulfoxyde ou (iv) l'eau (élimination par évaporation), utilisés seuls ou en mélange.

La couche A comprend, en outre, éventuellement :
- un agent de collage y, choisi parmi les polymères comportant à la fois au moins une séquence poly A compatible avec la couche A et au moins une séquence poly E compatible avec la couche E sélectionnés dans le groupe constitué par les copolymères di-blocs, de type polyA-bloc-polyE, les copolymères tri-blocs de type polyE-bloc-polyA-bloc-polyE (EAE), de type polyA-bloc-polyE-bloc-polyA (AEA), de type polyA-bloc-polyE-bloc-polyF (AEF), de type polyA-bloc-polyF-bloc-polyE (AFE), et les copolymères greffés de type polyA-greffé-polyE, polyE-greffé-polyA ou de type polyA-greffé-polyE et -polyF, la séquence -poly F pouvant être compatible avec la couche A ou avec la couche E ou parmi les adhésifs de type acrylique, silicone ou polyuréthane.

Les séquences poly A sont choisies dans le groupe qui comprend le polyoxyéthylène, la polyvinylpyridine, les poly(acides acryliques), le polyalcoolvinylique et la polyvinylpyridine quaternisée et les séquences poly E sont choisies dans le groupe qui comprend les polydiènes, les polyoléfines, le polyoxypropylène et le polydiméthylsiloxane.

A titre d'exemple de polydiènes ou de polyoléfines, on peut citer le polybutadiène, le polyisoprène, le polybutadiène hydrogéné, le polyisoprène hydrogéné, le polystyrène, le poly-4-tertiobutylstyrène.

Parmi les structures greffées comportant des séquences poly A, poly E et poly F, on peut notamment citer :
- en tant que séquences poly F, des chaînes acryliques par exemple,
- en tant que séquences poly E, des chaînes alkyles par exemple et
- en tant que séquences poly A, des chaînes polyoxyéthylène par exemple.

Ces structures correspondent à certains polymères greffés commercialisés par la société Goldschmidt sous le nom commercial "BRINOIL LE®".

Selon un autre mode de réalisation dudit matériau multicouche, ladite couche A de gel comprend :
- entre 0 et 98 %, de préférence entre 25 et 98 % de solvant sₐ par rapport au gel,
- entre 0,1 et 74 %, de préférence entre 1 et 74 % de polymère structurant c par rapport au gel,
- entre 1 et 80 %, de préférence entre 1 et 74 % de ladite substance chimique active x par rapport au gel et
- entre 0,0001 et 0,1 % de réticulant ri (teneur massique réticulant/ensemble des constituants).

Un agent de collage y peut être ajouté dans des proportions massiques de 0,01 à 25 % par rapport au gel ; les proportions de solvant sₐ sont adaptées en conséquence.

Egalement conformément à l'invention, ledit matériau multicouche comprend en tant que couche E, un élastomère de synthèse e, sélectionné, et ce de manière non limitative, parmi le polybutadiène, le polyisoprène, les polymères acryliques, le polychloroprène, le polyuréthane, les copolymères à base de chlorobutadiène et d'acide méthacrylique ou à base d'éthylène et d'acétate de vinyle, les copolymères SBR *(Styrene Butadiene Rubber),* SBS (Styrène Butadiène Styrène), SIS (Styrène Isoprène Styrène) ou SEBS (Styrène Ethylène Butylène Styrène) et éventuellement, un agent de collage y, tel que défini ci-dessus.

L'élastomère e peut être utilisé, soit sous forme d'une solution dans un solvant organique sₑ, soit sous forme d'une dispersion aqueuse (latex).

Egalement conformément à l'invention, ledit matériau multicouche comprend en tant que couche de collage Z, un polymère z, sélectionné dans le groupe constitué par (i) les copolymères di-bloc, tri-bloc ou greffés décrits dans les dispositions qui précèdent (copolymère utilisé en tant qu'agent de collage y), (ii) les polymères de type acrylique ou vinylique, comme par exemple l'acétate de vinyle ou le laurate de vinyle, (iii) les polymères dièniques comportant des fonctions nitriles, comme le NBR (*Nitrile Butadiene Rubber*), les polyuréthanes et certains polyesters ou polyamides et (iv) des copolymères du même type que ceux sélectionnés pour la couche E traités pour les rendre compatibles avec la couche A, soit chimiquement par greffage comme le SEBS traité à l'anhydride maléique ou par attaque chimique du SBS ou du SIS avec une solution d'acide sulfurique ou bien physiquement par bombardement de la surface du film à l'aide d'ions ou d'électrons : traitement Corona et Plasma ou de photons : traitement ultraviolet.

Le polymère z peut être utilisé, soit sous forme d'une solution dans un solvant organique s_{z}, soit sous forme d'une dispersion aqueuse (latex).

De tels films polymériques ou matériaux multicouches, formés d'au moins une couche continue A de gel, contenant éventuellement un agent de collage y, et d'au moins deux couches barrière E d'élastomère, contenant éventuellement un agent de collage y, solidarisées à l'aide dudit agent de collage y et/ou à l'aide d'une couche de collage Z indépendante, présentent, de manière inattendue, une structure souple et élastique, particulièrement bien adaptée à la préparation de gants, doigtiers ou préservatifs. Ces derniers assurent au porteur dudit matériau, notamment lors d'examens médicaux, d'interventions chirurgicales ou dans l'art dentaire, une protection efficace à l'encontre d'agents infectieux transmissibles par le sang, et ce notamment en cas de rupture ou même simplement de fissures de la membrane d'élastomère, qui pourraient entraîner une contamination du porteur dudit matériau, dont l'utilisation n'est donc pas sans risque en l'absence du matériau multicouche selon l'invention.

La présente invention a également pour objet des procédés de préparation d'un matériau multicouche selon l'invention comprenant au moins une couche A constituée d'un gel non-réversible, tel que défini ci-dessus et au moins deux couches barrière E, comprenant un élastomère de synthèse e, la solidarisation desdites couches entre elles étant assurée par un agent de collage polymérique y, incorporé dans au moins l'une desdites couches A ou E et/ou par une couche de collage Z indépendante, comprenant un polymère z :
- Le procédé ① « couche A gel non-réversible » comprend :
   1) la préparation d'une première couche barrière d'élastomère E, chargée ou non en agent de collage y, par dissolution d'un polymère e, conforme à l'invention, dans un solvant sₑ, tel que défini ci-dessus, étalement de ladite solution sur un support approprié et évaporation du solvant sₑ ; et éventuellement réticulation ;
   2) la préparation d'au moins une couche A selon le procédé suivant (incorporation du réticulant ri directement dans la solution) :
      - préparation d'une solution par mélange d'au moins une substance chimique active x, éventuellement dans un solvant sₐ, avec un polymère structurant c, et éventuellement un agent de collage y,
      - incorporation d'un réticulant ri, directement dans ladite solution,
      - étalement de ladite solution sur la couche obtenue à l'étape 1) (filmification),
      - initiation de la réticulation par voie photochimique et/ou thermique ou encore par contact avec un accélérateur de réticulation ;
   3) l'étalement d'une succession de couches A et E selon un ordre désiré et séparées ou non par des couches de collage Z, lesquelles couches A et E étant préparées conformément aux étapes 1) et 2) et
   4) la préparation et l'étalement d'une ultime couche barrière d'élastomère E, conformément à l'étape 1).
- Le procédé ② « couche A gel non-réversible » comprend :
   1) la préparation d'une première couche barrière d'élastomère E, conformément à l'étape 1) du procédé ① « couche A gel non-réversible » ;
   2) la préparation d'au moins une couche A selon le procédé suivant (introduction du réticulant ri simultanément au mélange) :
      - préparation d'une solution, par mélange d'au moins une substance chimique active x, éventuellement dans un solvant sₐ, avec un polymère structurant c, et éventuellement un agent de collage y,
      - introduction simultanément au mélange comprenant le polymère c, la substance chimique active x, le solvant sₐ et éventuellement l'agent de collage y, d'un réticulant ri, par exemple par pulvérisation ; la réticulation a lieu rapidement et induit la formation du film ;
      - étalement de ladite solution sur la couche obtenue à l'étape 1), suivie d'une réticulation rapide, induisant la formation du film ;
   3) l'étalement d'une succession de couches A et E selon un ordre désiré et séparées ou non par des couches de collage Z, lesquelles couches A et E étant préparées conformément aux étapes 1) et 2) et
   4) la préparation et l'étalement d'une ultime couche barrière d'élastomère E conformément à l'étape 1).

Conformément à l'invention, la superposition de différents films A réalisés avec diverses substances chimiques actives x permet de cumuler les effets desdites substances chimiques sans les associer.

Selon un mode de réalisation avantageux desdits procédés, la couche de collage Z est préparée par évaporation du solvant s_{z} ou de l'eau à partir d'un mélange comprenant un polymère z, tel que défini ci-dessus, en solution dans un solvant s_{z}, sélectionné dans le groupe qui comprend les hydrocarbures aromatiques, aliphatiques et alicycliques, les composés plus polaires comme les éthers tels le tétrahydrofurane, les cétones, le diméthyl formamide, le diméthyl sulfoxyde ou les alcools, ainsi que l'eau ou un mélange de polarité intermédiaire.

Selon une disposition avantageuse de ce mode de réalisation, la couche de collage Z subit une réticulation initiée de manière photochimique, comme par exemple sous l'action du rayonnement ultra-violet et accélérée de façon thermique ou encore chimique, comme par exemple par oxydation ou à l'aide d'agent de réticulation. Le solvant est de l'eau dans le cas où le polymère z est utilisé sous la forme d'une dispersion (latex).

En variante, dans le cas où la solidarisation desdites couches entre elles est assurée par un traitement chimique ou physique, le procédé de préparation dudit matériau multicouche comprend, après l'étalement d'une couche A et/ou d'une couche E, sur un support approprié, un traitement chimique ou physique, tel que défini ci-dessus.

Egalement en variante, le matériau multicouche selon l'invention comprenant en tant que couche A, un gel non-réversible, comprend une étape de préparation du polymère structurant c par polymérisation *in situ* à partir d'un mélange d'un ou plusieurs monomères m et d'au moins une substance chimique active x, par voie thermique, par voie photochimique (rayonnement ultraviolet) ou radiochimique (source de cobalt ⁶⁰Co).

Selon un mode de mise en oeuvre avantageux de cette variante, ledit mélange comprend en outre au moins l'un des composants additionnels suivants : un initiateur i, un ou plusieurs solvants sₐ, un épaississant f, un réticulant ri, un agent de collage y.

Selon une disposition avantageuse de ce mode de mise en oeuvre, lesdits monomères m sont sélectionnés de préférence dans le groupe constitué par les acrylates et leurs dérivés, comme l'acide acrylique, l'hydroxyéthyl méthacrylate, le méthacrylate de méthyle, les acrylamides, les acrylamines et leur dérivés, la vinyl pyrrolidone.

Selon une modalité avantageuse de cette disposition, le nombre de monomères utilisé est compris entre 1 et 10, de préférence entre 1 et 4.

Conformément à cette variante :
- dans le cas d'une polymérisation initiée par voie thermique, l'initateur i est sélectionné dans le groupe constitué par la famille des peroxydes comme le peroxyde de benzoyle, la famille des azonitriles comme l'azobisbutyronitrile ou les couples redox comme le couple S₂O₈/S₂O₅ ;
- dans le cas d'une polymérisation initiée par voie photochimique, l'initateur i est sélectionné dans le groupe constitué par la famille des acétophénones, les dérivés de la benzoïne ou la famille des peroxydes.

Selon une autre disposition avantageuse de ce mode de mise en oeuvre, l'épaississant f est sélectionné dans le groupe constitué par les stéarates d'aluminium, les pâtes de linoléate de chaux, l'huile de ricin hydrogénée, les triglycérides, les argiles modifiées de type bentonite, les esters de polyols, les silices et les polymères compatibles avec le mélange de monomères ou de même nature que le polymère gélifié comme certains polyacides acryliques modifiés, les polyméthacrylates ou la polyvinyl pyrrolidone.

La présente invention a également pour objet les différentes applications du film d'élastomère selon l'invention en tant que revêtement de supports notamment en élastomère ou en plastique (gants, doigtiers, préservatifs..) ou de surmoulage d'un joint frottant.

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés, dans lesquels :

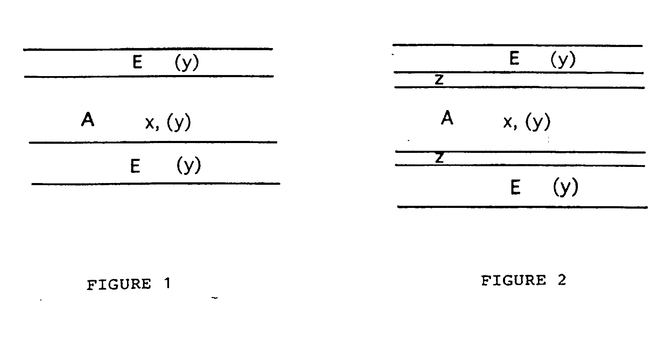

- les figures 1 à 4 illustrent différents modes de réalisation du matériau multicouche selon l'invention, dans lesquelles les abréviations c, e, x, ri, sₐ, sₑ, s_{z}, y, z ont les significations suivantes : c : polymère structurant c ; e : élastomère e ; x : substance chimique active x ; ri : réticulant irréversible ; y : agent de collage y ; z : polymère z assurant l'adhésion entre deux couches de structures différentes et dans lesquelles les parenthèses signifient que le produit est optionnellement présent.

Il doit être bien entendu, toutefois, que ces exemples sont donnés uniquement à titre d'illustration de l'objet de l'invention, dont ils ne constituent en aucune manière une limitation.

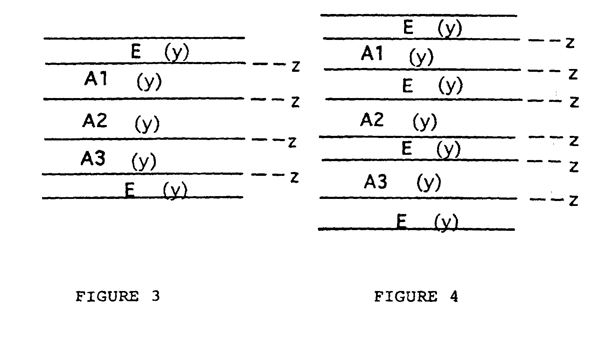

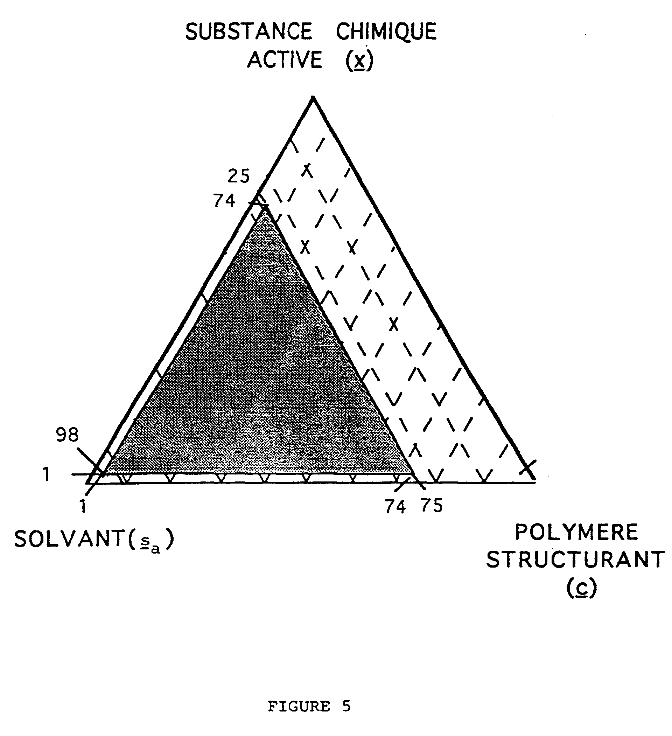

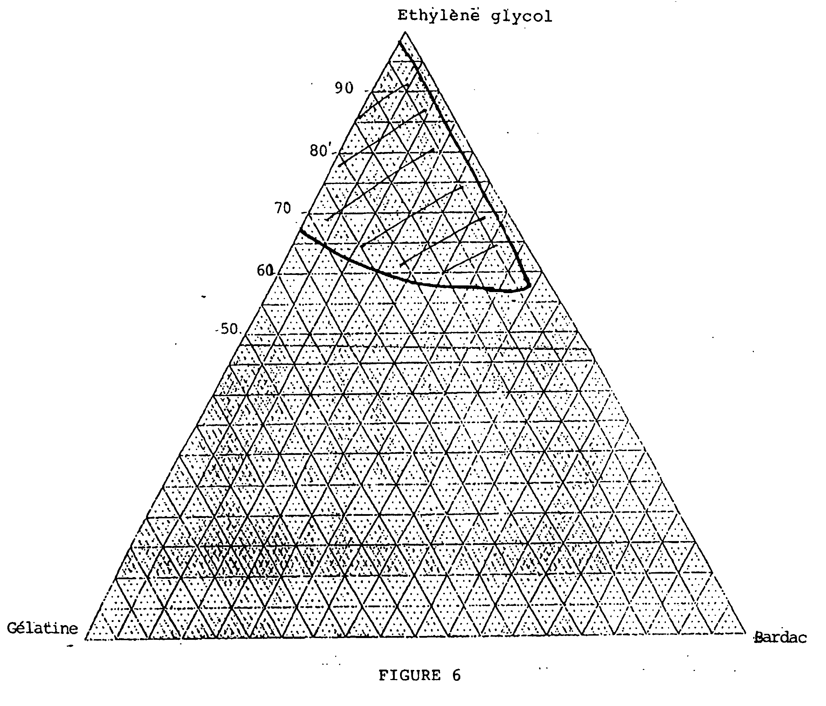

Conformément à l'invention un tel film polymérique multicouche, constitué d'une superposition de couches peut avantageusement présenter, et ce, de manière non limitative, l'une des structures suivantes :
- film polymérique à trois couches comprenant une couche A en sandwich entre deux couches E (figure 1) ;
- film polymérique à cinq couches comprenant successivement une couche E, une couche Z, une couche A, une couche Z et une couche E (figure 2) ;
- film polymérique à cinq couches comprenant successivement une couche E, une couche A1, une couche A2, une couche A3 et une couche E (figure 3) ;
- film polymérique à 9 couches comprenant successivement une couche E, une couche Z, une couche A1, une couche Z une couche A2, une couche Z une couche A3, une couche Z et une couche E (figure 3) ;
- film polymérique à 7 couches comprenant successivement une couche E, une couche A1, une couche E, une couche A2, une couche E, une couche A3 et une couche E (figure 4) ;
- film polymérique à 13 couches comprenant successivement une couche E, une couche Z, une couche A1, une couche Z, une couche E, une couche Z, une couche A2, une couche Z, une couche E, une couche Z, une couche A3, une couche Z et une couche E (figure 4).

Dans l'ensemble de ces structures, aussi bien les couches A que les couches E peuvent, optionnellement contenir un agent de collage y , alors que les couches A peuvent, en outre, optionnellement comprendre un réticulant ri (représentés respectivement par (y) et (ri) sur les figures).

Les couches A1, A2 et A3 peuvent comporter des concentrations différentes des mêmes substances chimiques actives x ou bien comporter chacune des substances chimiques actives x de nature différente.

### EXEMPLE 1 : Préparation d'une couche A réticulée de manière non réversible.

On dissout, sous agitation et à 60°C de la gélatine issue par traitement acide du collagène et d'indice de Bloom de 175, dans un mélange de chlorure de didécyldiméthylammonium commercialisé par la société LONZA sous le nom de Bardac® 2270E (soit Bardac®⁾ et d'éthylène glycol (ou de l'eau) comprenant 10 % en masse de Bardac® par rapport au gel.

La solution limpide formée contient 15 % en masse en gélatine.

La température du milieu (60°C) correspond à une température supérieure à la température de transition gel-liquide, qui est déterminée par variation brusque de la viscosité du mélange à cette température et est, dans le cas présent de 30-35°C.

Le film A s'obtient par refroidissement de cette solution, préalablement conditionnée sous forme d'un film à 50°C à l'aide d'un tire-film d'épaisseur contrôlée de 100 µm ou par refroidissement de cette solution sur une forme en céramique, selon le principe du trempé.

A la couche A ainsi obtenue, on ajoute 3 % de glutaraldéhyde.

### EXEMPLE 2 : Préparation d'une couche A réticulée par des rayons γ.

On prépare une solution à 8 % de PVP dans l'eau que l'on verse entre deux plaques de verre espacées de 1 mm. Le système est placé à 31,5 cm d'une source de ⁶⁰Co d'intensité 0,66 rad/min, durant un mois. Le gel ainsi obtenu possède une bonne souplesse.

### EXEMPLE 3 : Préparation d'une couche A réticulée de manière non réversible.

Dans cet exemple, le polymère structurant c est de l'alcool polyvinylique de type Mowiol® 28-99, caractérisé par une masse moléculaire de 88000 Daltons et un pourcentage d'hydrolyse de 99 %. Sa viscosité, en solution à 4 % dans l'eau et à 20°C, est de 28 mPa.s.

On dissout sous agitation et à 80°C du Mowiol® dans de l'eau (solvant sₐ) de telle façon à obtenir une solution à 2,5 % de Mowiol®.

A cette solution est rajouté, sous agitation, du Triton X100® (substance chimique active jusqu'à obtenir une solution limpide contenant 10 % de Triton (exprimé par rapport à la solution de Mowiol®).

La solution limpide obtenue se présente sous la forme d'un liquide visqueux.

La solution contenant le réticulant irréversible ri se prépare en mélangeant du glutaraldéhyde dans un mélange eau : acide chlorhydrique comprenant 9 parties d'eau pour une partie d'acide molaire, de telle façon à obtenir une solution à 0,005 % en glutaraldéhyde.

La préparation de la couche A se réalise par pulvérisation simultanément de 10 parties massiques de la solution contenant le Mowiol® avec 0,0475 partie massique de la solution contenant le réticulant irréversible.

La couche A ainsi réalisée présente une élasticité et une souplesse acceptable.

### EXEMPLE 4 : Préparation d'un multimatériau à 5 couches dont la couche A est obtenue par polymérisation in situ, en présence d'un épaississant f et en présence d'une couche de collage Z.

La couche barrière E est obtenue par trempé d'une forme dans une solution à 15 % en masse en PI obtenue par dissolution, sous agitation, du polyisoprène (PI) dans du cyclohexane.

La forme est alors trempée dans une solution de copolymère éthylène/acide acrylique à 3 % dans du toluène commercialisé sous le nom de Vamac® par la société Dupont ou ATX® par la société Exxon, puis dans une solution contenant de l'hydroxyéthyl méthacrylate (HEMA), du Bardac®, du PEG dans les proportions 2/1/1, la polymérisation étant ensuite initiée à 70°C en présence du POB (peroxyde de benzoyle) et du poly(acide acrylique) commercialisé par la société Protex sous le nom de Modarez 200.

Après polymérisation, la forme est trempée dans une solution d'élastomère e comme décrit précédemment.

### EXEMPLE 5 : Préparation d'un multimatériau à 5 couches dont la couche A est obtenue par polymérisation in situ et comprenant une couche de collage Z.

La couche barrière E est obtenue par le même procédé que celui de l'exemple 4. Sur cette couche est ajoutée, selon le même procédé, une couche de collage composée d'un latex acrylique commercialisé sous le nom de Primal EP 6010K. Après séchage, la couche A est déposée selon le principe du trempé. Elle résulte du mélange de 0 à 50 % de méthacrylate de méthyle avec 100 à 50 % d'hydroxyéthyl méthacrylate en présence d'eau, de Bardac®, de PEG 400.

L'initiation est réalisée avec du POB à 70°C. Après obtention du gel, la forme est de nouveau plongée dans la solution de Primal® avant d'être recouverte d'une seconde couche barrière E comme décrit dans l'exemple 4.

### EXEMPLE 6 : Préparation d'un multimatériau à 3 couches dont la couche A est obtenue par polymérisation in situ initiée par les UV.

La couche barrière E est obtenue par le même procédé que celui de l'exemple 4. Sur cette couche est ajoutée selon le même procédé, un mélange HEMA/NVP (N-vinylpyrrolidone) (70/30) en présence d'un initiateur i : 2-hydroxy-2,2-diméthyl acétophénone (Darocur® 1173) et d'un solvant sₐ : glycérol.

La polymérisation est initiée par rayonnement UV (λ = 365 nm) pendant 2 heures. Après obtention du gel, la forme est trempée dans une solution d'élastomère E comme décrit précédemment.

### EXEMPLE 7 : Préparation d'un multimatériau à 4 couches possédant une couche A réticulée de manière non réversible et dont l'adhésion intercouche est assurée par un traitement de la couche d'élastomère E.

Cet exemple traite de la réalisation d'un matériau multicouche constituée d'une couche A insérée entre deux couches barrière E et dont l'adhésion est assurée par un traitement plasma de la couche d'élastomère E d'un côté et par une couche de collage Z de l'autre côté.

La couche d'élastomère E est obtenue par trempé d'une forme céramique dans une solution à 15 % de SBS ou de SIS, conformément à l'exemple 4.

La forme est placée dans une enceinte dans laquelle on fait passer un gaz ionisé d'azote, d'argon ou d'oxygène de façon à oxyder la surface. L'amélioration de la mouillabilité de l'élastomère assure une bonne adhésion avec la couche obtenue comme dans l'exemple 4. La forme est alors plongée dans une solution comprenant de la gélatine, du Bardac® et de l'éthylène glycol (comme décrit dans l'exemple 4), puis dans une solution à 0,3 % de glutaraldéhyde dans l'acétone, puis recouverte d'une couche d'élastomère E comme décrit précédemment.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Matériau polymérique multicouche, **caractérisé en ce qu'**il comprend au moins une couche A constituée d'un gel non-réversible en fonction de la température, et chargé en au moins une substance chimique active x et au moins deux couches barrière E, comprenant un élastomère de synthèse e, la solidarisation desdites couches entre elles étant assurée par un agent de collage polymérique y, incorporé dans au moins l'une desdites couches A ou E et/ou par une couche de collage Z indépendante, comprenant un polymère z et/ou un traitement chimique ou physique d'au moins l'une desdites couches A ou E.

2. Matériau multicouche selon la revendication 1, **caractérisé en ce que** le gel non-réversible comprend essentiellement un polymère structurant c, un réticulant ri, au moins une substance chimique active x et un solvant sₐ.

3. Matériau multicouche selon la revendication 2, **caractérisé en ce que** ledit gel non-réversible contient, en outre, un agent de collage y.

4. Matériau multicouche selon l'une quelconque des revendications 2 ou 3, **caractérisé en ce que** le polymère structurant c de la couche A est un polymère non miscible avec l'élastomère de synthèse e,

5. Matériau multicouche selon l'une quelconque des revendications 2 à 4, **caractérisé en ce que** le polymère structurant c de la couche A est de type hydrophile et compatible avec la substance chimique active x, seule ou en solution dans un solvant sₐ.

6. Matériau multicouche selon la revendication 2 ou la revendication 3, **caractérisé en ce que** ledit polymère structurant c de la couche A est sélectionné dans le groupe constitué par (i) les polymères d'origine naturelle choisis parmi la gélatine, les gommes, les pectines et les alginates ; les polypeptides ; les polyurées ; les héparinoïdes et le polyacide gluconique ; et les dérivés cellulosiques choisis parmi la méthylcellulose, l'hydroxyéthylcellulose, l'hydroxypropylcellulose et la carboxyméthylcellulose et (ii) les polymères de synthèse choisis parmi le polyéthyleneimine, l'acide polyacrylique, l'alcool polyvinylique, seuls ou en réseaux interpénétrés, les polyacrylamides et leurs dérivés, la polyvinyl pyrrolidone, le polydiméthyl siloxane et les poly(vinyléthers).

7. Matériau multicouche selon la revendication 2, **caractérisé en ce que** le réticulant ri de la couche A est sélectionné, pour les polymères c comportant des fonctions hydroxyles, parmi les aldéhydes ou dialdéhydes à faible ou à haut poids moléculaire et les dérivés urée formol ou mélamine formol, et pour les polymères comportant des fonctions hydroxyles, carboxyliques ou amines, parmi des prépolymères comportant des fonctions isocyanates ou parmi les méthacrylates, les imides ou les persulfates.

8. Matériau multicouche selon l'une quelconque des revendications 1 à 7, **caractérisé en ce que** ladite substance chimique active x présente dans ladite couche A est sélectionnée parmi les composés étant capables de provoquer une dénaturation quasi-instantanée des protéines par simple contact, soit par réaction chimique ou par effet physicochimique comme par modification de la tension de surface.

9. Matériau multicouche selon la revendication 8, **caractérisé en ce que** ladite substance chimique x est sélectionnée parmi les biocides.

10. Matériau multicouche selon la revendication 9, **caractérisé en ce que** ledit biocide est sélectionné dans le groupe constitué par les ammonium quaternaires, des biguanides, le phtalaldéhyde, des dérivés phénoliques, le formol, des tensio-actifs non ioniques comportant au moins une séquence polyoxyéthylène, l'hexamidine, des composés iodés, des tensio-actifs non ioniques à activité virucide, les bichromates et hypochlorites de sodium et de potassium ou des polymères chargés comme les polyélectrolytes présentant des activités biocides comme le poly(acide acrylique), le polychlorure de triméthylammonium de divinyl benzyle, le copolymère de divinyl éther-anhydride maléique, les polyéthylène imines, les polyéther amines, les polyurées et les polypeptides, utilisés seuls ou en mélange.

11. Matériau multicouche selon la revendication 10, **caractérisé en ce que** ledit biocide est le diméthyldidécylammonium.

12. Matériau multicouche selon la revendication 2, **caractérisé en ce que** le solvant sₐ de la couche A est sélectionné dans le groupe constitué par (i) les composés peu volatils choisi parmi les polyols, (ii) l'eau et (iii) des solvants volatils tels que les alcools et les cétones à faible poids moléculaire, les composés éthérés non cycliques ou cycliques, le diméthylformamide, le diméthyl sulfoxyde, utilisés seuls ou en mélange.

13. Matériau multicouche selon la revendication 1, **caractérisé en ce que** l'agent de collage y est choisi parmi les polymères comportant à la fois au moins une séquence poly A compatible avec la couche A et au moins une séquence poly E compatible avec la couche E sélectionnés dans le groupe constitué par les copolymères di-blocs, de type polyA-bloc-polyE, les copolymères tri-blocs de type polyE-bloc-polyA-bloc-polyE (EAE), de type polyA-bloc-polyE-bloc-polyA (AEA), de type polyA-bloc-polyE-bloc-polyF (AEF), de type polyA-bloc-polyF-bloc-polyE (AFE), et les copolymères greffés de type polyA-greffé-polyE, polyE-greffé-polyA ou de type polyA-greffé-polyE et -polyF, la séquence -poly F étant compatible avec la couche A ou avec la couche E ou parmi les adhésifs de type acrylique, silicone ou polyuréthane.

14. Matériau multicouche selon la revendication 13, **caractérisé en ce que** les séquences poly A sont choisies dans le groupe qui comprend le polyoxyéthylène, la polyvinylpyridine, les polyacides acryliques, le polyalcoolvinylique et la polyvinylpyridine quatemisée et les séquences poly E sont choisies dans le groupe qui comprend les polydiènes, les polyoléfines, le polyoxypropylène et le polydiméthylsiloxane.

15. Matériau multicouche selon l'une quelconque des revendications 1 à 7, **caractérisé en ce que** ladite couche A de gel comprend:
- entre 0 et 98 % de solvant sₐ par rapport au gel
- entre 0,1 et 74 % de polymère structurant c par rapport au gel
- entre 1 et 80 % de ladite substance chimique active x par rapport au gel et
- entre 0,0001 et 0,1 % de réticulant ri (teneur massique réticulant/ensemble des constituants).

16. Matériau multicouche selon la revendication 15, **caractérisé en ce que** ladite couche A de gel comprend :
- entre 25 et 98 % de solvant sₐ par rapport au gel
- entre 1 et 74 % de polymère structurant c par rapport au gel
- entre 1 et 74 % de ladite substance chimique active x par rapport au gel.

17. Matériau multicouche selon la revendication 15, **caractérisé en ce que** lorsque ladite couche A de gel comprend en outre un agent de collage y, les proportions massiques de ce dernier sont comprises entre 0,01 et 25 % par rapport au gel, les proportions de solvant sₐ étant adaptées en conséquence.

18. Matériau multicouche selon l'une quelconque des revendications 1 à 17, **caractérisé en ce que** les couches d'élastomère E comprennent un élastomère de synthèse e sélectionné parmi le polybutadiène, le polyisoprène, les polymères acryliques, le polychloroprène, le polyuréthane, les copolymères à base de chlorobutadiène et d'acide méthacrylique ou à base d'éthylène et d'acétate de vinyle, les copolymères SBR (*Styrene Butadiene Rubber*), SBS (Styrène Butadiène Styrène), SIS (Styrène Isoprène Styrène) ou SEBS (Styrène Ethylène Butylène Styrène) et éventuellement un agent de collage y, tel que défini à la revendication 13 ou à la revendication 14.

19. Matériau multicouche selon la revendication 1, **caractérisé en ce que** le polymère z de la couche de collage Z est sélectionné dans le groupe constitué par (i) les copolymères di-bloc, tri-bloc ou greffés selon la revendication 13, (ii) les polymères de type acrylique ou vinylique, (iii) les polymères dièniques comportant des fonctions nitriles comme le NBR (*Nitrile Butadiene Rubber),* les polyuréthanes et certains polyesters ou polyamides et (iv) des copolymères du même type que ceux sélectionnés pour la couche E traités pour les rendre compatibles avec la couche A, soit chimiquement par greffage ou par attaque chimique avec une solution d'acide ou bien physiquement par bombardement de la surface du film à l'aide d'ions, d'électrons ou de photons.

20. Procédé de préparation d'un matériau multicouche comprenant au moins une couche A constituée d'un gel non-réversible selon la revendication 1, **caractérisé en ce qu'**il comprend :
1) la préparation d'une première couche barrière d'élastomère E, chargée ou non en agent de collage y, par dissolution d'un polymère e, tel que défini à la revendication 18 dans un solvant sₑ, sélectionné dans le groupe constitué par (i) les hydrocarbures aromatiques, aliphatiques et alicycliques, par exemple des hydrocarbures paraffiniques, le cyclohexane, le benzène, le toluène, le xylène, la tétraline, la décaline, (ii) des coupes pétrolières, (iii) d'autres solvants plus polaires tels que des alcools ou les cétones à faible poids moléculaire, (iv) des composés éthérés non cycliques ou cycliques, (v) de l'eau, (vi) le diméthyl formamide ou le diméthyl sulfoxyde, utilisés seuls ou en mélanges, étalement de ladite solution sur un support approprié et évaporation du solvant sₑ ; et éventuellement réticulation ;
2) la préparation d'au moins une couche A selon le procédé suivant :
- préparation d'une solution par mélange d'au moins une substance chimique active x, éventuellement dans un solvant sₐ, avec un polymère structurant c, et éventuellement un agent de collage y,
- incorporation d'un réticulant ri, directement dans ladite solution,
- étalement de ladite solution sur la couche obtenue à l'étape 1),
- initiation de la réticulation par voie photochimique et/ou thermique ou encore par contact avec un accélérateur de réticulation ;
3) l'étalement d'une succession de couches A et E selon un ordre désiré et séparées ou non par des couches de collage Z, lesquelles couches A et E étant préparées conformément aux étapes 1) et 2) et
4) la préparation et l'étalement d'une ultime couche barrière d'élastomère E chargée ou non en agent de collage y, par évaporation du solvant sₑ ou de l'eau, cette étape étant éventuellement suivie par une étape de réticulation.

21. Procédé de préparation d'un matériau multicouche comprenant au moins une couche A constituée d'un gel non-réversible selon la revendication 1, **caractérisé en ce qu'**il comprend :
1) la préparation d'une première couche barrière d'élastomère E, chargée ou non en agent de collage y, par dissolution d'un polymère e, tel que défini à la revendication 18 dans un solvant se, tel que défini à la revendication 20, étalement de ladite solution sur un support approprié et évaporation du solvant sₑ ; et éventuellement réticulation ;
2) la préparation d'au moins une couche A selon le procédé suivant :
- préparation d'une solution par mélange d'au moins une substance chimique active x, éventuellement dans un solvant sₐ, avec un polymère structurant c, et éventuellement un agent de collage y,
- introduction simultanément au mélange comprenant le polymère c, la substance chimique active x, le solvant sₐ et éventuellement l'agent de collage y, d'un réticulant ri,
- étalement de ladite solution sur la couche obtenue à l'étape 1) suivie d'une réticulation rapide, induisant la formation du film ;
3) l'étalement d'une succession de couches A et E selon un ordre désiré et séparées ou non par des couches de collage Z, lesquelles couches A et E étant préparées conformément aux étapes 1) et 2) et
4) la préparation et l'étalement d'une ultime couche barrière d'élastomère E chargée ou non en agent de collage y par évaporation du solvant sₑ ou de l'eau, cette étape étant éventuellement suivie par une étape de réticulation.

22. Procédé de préparation selon la revendication 20 ou 21, **caractérisé en ce que** la couche de collage Z est préparée par évaporation du solvant s_{z} ou de l'eau à partir d'un mélange comprenant un polymère z selon la revendication 19, en solution dans un solvant s_{z}, sélectionné dans le groupe qui comprend les hydrocarbures aromatiques, aliphatiques et alicycliques, les composés plus polaires comme les éthers tels le tétrahydrofurane, le diméthyl formamide, le diméthyl sulfoxyde ou les cétones, les alcools, ainsi que l'eau ou un mélange de polarité intermédiaire.

23. Procédé de préparation selon la revendication 22, **caractérisé en ce que** la couche de collage Z subit une réticulation initiée de manière photochimique et accélérée de façon thermique ou encore chimique.

24. Procédé de préparation d'un matériau multicouche comprenant en tant que couche A, un gel non-réversible selon la revendication 2, **caractérisé en ce qu'**il comprend une étape de préparation du polymère structurant c par polymérisation *in situ* à partir d'un mélange d'un ou plusieurs monomères m et d'au moins une substance chimique active x, par voie thermique, photochimique ou radiochimique.

25. Procédé selon la revendication 24, **caractérisé en ce que** ledit mélange comprend en outre au moins l'un des composants additionnels suivants : un initiateur i, un ou plusieurs solvants sₐ, un épaississant f, un réticulant ri, un agent de collage y.

26. Procédé selon la revendication 24, **caractérisé en ce que** lesdits monomères m sont sélectionnés dans le groupe constitué par les acryliques, les acrylates, les acrylamides, les acrylamines et leur dérivés, la vinyl pyrrolidone.

27. Procédé selon la revendication 24, **caractérisé en ce que** le nombre de monomères utilisé est compris entre 1 et 10, de préférence entre 1 et 4.

28. Procédé selon la revendication 24 ou la revendication 25, **caractérisé en ce que** dans le cas d'une polymérisation initiée par voie thermique, l'initateur i est sélectionné dans le groupe constitué par la famille des peroxydes comme le peroxyde de benzoyle, la famille des azonitriles comme l'azobisbutyronitrile ou les couples redox comme le couple S₂O₈/S₂O₅ .

29. Procédé selon la revendication 24 ou la revendication 25, **caractérisé en ce que** dans le cas d'une polymérisation initiée par voie photochimique, l'initateur i est sélectionné dans le groupe constitué par la famille des acétophénones, les dérivés de la benzoïne ou la famille des peroxydes.

30. Procédé selon la revendication 24 ou la revendication 25, **caractérisé en ce que** l'épaississant f est sélectionné dans le groupe constitué par les stéarates d'aluminium, les pâtes de linoléate de chaux, l'huile de ricin hydrogénée, les triglycérides, les argiles modifiées de type bentonite, les esters de polyols, les silices et les polymères compatibles avec le mélange de monomères ou de même nature que le polymère gélifié.

31. Procédé de préparation d'un matériau multicouche selon la revendication 1, **caractérisé en ce qu'**il comprend des étapes de préparation des couches A et E selon les revendications 20 à 30, un traitement chimique ou physique desdites couches A et/ou E étant effectué après leur étalement sur un support approprié.

32. Procédé selon la revendication 31, **caractérisé en ce que** ledit traitement chimique est effectué par greffage ou par attaque chimique avec une solution d'acide et le traitement physique est effectué par bombardement de la surface du film à l'aide d'ions, d'électrons ou de photons.

33. Applications du matériau multicouche selon l'une quelconque des revendications 1 à 19 en tant que revêtement de supports notamment en élastomère ou en plastique ou de surmoulage d'un joint frottant.

34. Gant, **caractérisé en ce qu'**il est revêtu d'un matériau multicouche selon l'une quelconque des revendications 1 à 19.

35. Doigtier, **caractérisé en ce qu'**il est revêtu d'un matériau multicouche selon l'une quelconque des revendications 1 à 19.

36. Préservatif, **caractérisé en ce qu'**il est revêtu d'un matériau multicouche selon l'une quelconque des revendications 1 à 19.

## Claims

1. A multilayer polymer material **characterized in that** it comprises at least one layer A composed of a gel which is irreversible as a function of the temperature and which comprises at least one active chemical substance x, and at least two barrier layers E comprising a synthetic elastomer e, the said layers A and E being held together by a polymeric bonding agent y incorporated in at least one of the said layers A or E and/or by an independent bonding layer Z comprising a polymer z and/or by a chemical or physical treatment of at least one of the said layers A or E.

2. Multilayer material according to claim 1, **characterized in that** the gel which is irreversible essentially comprises a structuring polymer c, a crosslinking agent ri, at least one active chemical substance x and a solvent sₐ.

3. Multilayer material according to claim 2, **characterized in that** the said irreversible gel contains, in addition, a bonding agent y.

4. Multilayer material according to any one of claims 2 or 3, **characterized in that** the structuring polymer c of the layer A is a polymer which is immiscible with the synthetic elastomer e.

5. Multilayer material according to any one of claims 2 to 4, **characterized in that** the structuring polymer c of the layer A is of hydrophilic type and compatible with the active chemical substance x, alone or in solution in a solvent sₐ.

6. Multilayer material according to claim 2 or to claim 3, **characterized in that** said structuring polymer c of the layer A is selected from the group consisting of (i) polymers of natural origin chosen among gelatin, gums, pectins and alginates; polypeptides; polyureas; heparinoids and poly(gluconic acid); cellulose derivatives chosen among methyl cellulose, hydroxyethyl cellulose, hydroxypropyl cellulose and carboxymethyl cellulose and (ii) synthetic polymers chosen among polyethyleneimine, poly(acrylic acid), polyvinyl alcohol, which are alone or as interpenetrated networks, polyacrylamides and their derivatives, polyvinylpyrrolidone, polydimethylsiloxane and poly(vinyl ethers).

7. Multilayer material according to claim 2, wherein the crosslinking agent ic of the layer A is selected, for the polymers c comprising hydroxyl functional groups, from aldehydes or dialdehydes of low or high molecular weight and from urea-formaldehyde or melamine-formaldehyde derivatives and, for the polymers containing hydroxyl, carboxyl or amine functional groups, from prepolymers containing isocyanate functional groups or from methacrylates, imides or persulfates.

8. Multilayer material according to any one of claims 1 to 7, **characterized in that** the said active chemical substance x present in the said layer A is selected from compounds capable of causing virtually instantaneous denaturation of proteins upon simple contact, either by chemical reaction or by physicochemical effect, such as by a modification of the surface tension.

9. Multilayer material according to claim 8, **characterized in that** said chemical substance x is selected from biocides.

10. Multilayer material according to claim 9, **characterized in that** the said biocide is selected from the group consisting of quaternary ammoniums, biguanides, phthalaldehyde, phenol derivatives, formaldehyde, non-ionic surfactants containing at least one polyoxyethylene sequence, hexamidine, iodinated compounds, non-ionic surfactants with a virucidal activity, sodium and potassium dichromates and hypochlorites or charged polymers such as polyelectrolytes exhibiting biocidal activities such as poly(acrylic acid), poly(divinylbenzyltrimethylammonium chloride), the divinyl ether-maleic anhydride copolymer, polyethyleneimines, polyetheramines, polyureas and polypeptides, employed alone or as a mixture.

11. Multilayer material according to claim 10, **characterized in that** the said biocide is dimethyldidecylammonium.

12. Multilayer material according to claim 2, **characterized in that** the solvent sₐ, for the layer A is selected from the group consisting of (i) compounds of low volatily chosen from polyols, (ii) water and (iii) volatile solvents such as alcohols and ketones of low molecular weight, non-cyclic and cyclic ether compounds, dimethylformamide, dimethylsulfoxide, employed alone or as a mixture.

13. Multilayer material according to claim 1, **characterized in that** the bonding agent y is chosen from polymers containing both at least one poly A sequence compatible with the layer A and at least one poly E sequence compatible with the layer E, which are selected from the group consisting of di-block copolymers of formula polyA-block-poly E, tri-block copolymers of formulas polyE-block-polyA-block-poly E (EAE), polyA-block-polyE-block-polyA (AEA), polyA-block-polyE-block-polyF (AEF) or polyA-block-polyF-block-polyE (AFE), and grafted copolymers of formulas polyA-grafted-polyE, polyE-grafted-polyA, or polyA-grafted-polyE and -polyF, the poly F sequence being compatible with the layer A or with the layer E, and from adhesives of acrylic, silicone or polyurethane type.

14. Multilayer material according to claim 13, **characterized in that** the poly A sequences are chosen from the group which comprises polyoxyethylene, polyvinylpyridine, poly(acrylic acid)s, polyvinyl alcohol and quaternized polyvinylpyridine and the poly E sequences are chosen from the group which comprises polydienes, polyolefins, polyoxypropylene and polydimethylsiloxane.

15. Multilayer material according to any one of claims 1 to 7, **characterized in that** the said gel layer A comprises:
- between 0 and 98% of solvent sₐ with respect to the gel,
- between 0.1 and 74% of structuring polymer c with respect to the gel,
- between 1 and 80% of the said active chemical substance x with respect to the gel, and
- between 0.0001 and 0.1% of crosslinking agent ic (content by mass/ combined constituents).

16. Multilayer material according to claim 15, **characterized in that** the said gel layer A comprises:
- between 25 and 98% of solvent sₐ with respect to the gel,
- between 1 and 74% of structuring polymer c with respect to the gel,
- between 1 and 74% of the said active chemical substance x with respect to the gel.

17. Multilayer material according to claim 15, **characterized in that**, when the said gel layer A additionally comprises a bonding agent y, the proportions by mass of the latter are between 0.01 and 25% with respect to the gel, the proportions of solvent sₐ being consequently adjusted.

18. Multilayer material according to any one of claims 1 to 17, **characterized in that** the elastomer layers E comprise a synthetic elastomer e, selected from polybutadiene, polyisoprene, acrylic polymers, polychloroprene, polyurethane, copolymers based on chlorobutadiene and methacrylic acid or based on ethylene and vinyl acetate, SBR copolymers (styrene butadiene rubber), SBS copolymers (styrene-butadiene-styrene), SIS copolymers (styrene-isoprene-styrene), SEBS copolymers (styrene-ethylene-butylene-styrene), and optionally a bonding agent y as defined in claim 13 or in claim 14.

19. Multilayer material according to claim 1, **characterized in that** the polymer z of the bonding layer Z is selected from the group consisting of (i) the di-block, tri-block or grafted copolymers according to claim 13, (ii) polymers of acrylic or vinyl type, (iii) diene polymers containing nitrile functional groups, such as NBR (nitrile-butadiene-rubber), polyurethanes and certains polyesters or polyamides and (iv) copolymers of the same type as those selected for the layers E, treated in order to render them compatible with the layer A, either chemically, by grafting or by chemical attack with an acid solution, or alternatively physically, by bombardment of the surface of the film using ions, electrons or photons.

20. Process for the preparation of a multilayer material comprising at least one layer A composed of a non-reversible gel according to claim 1, **characterized in that** it comprises:
1) the preparation of a first elastomer barrier layer E, which may or may not carry a bonding agent y, by dissolution of a polymer e, as defined in claim 18, in a solvent sₑ, selected from the group consisting of: (i) aromatic, aliphatic and alicyclic hydrocarbons, for example paraffin hydrocarbons, cyclohexane, benzene, toluene, xylene, tetralin, decalin, (ii) petroleum fractions, (iii) other more polar solvents, such as alcohols or ketones of low molecular weight, (iv) non-cyclic or cyclic ether compounds, (v) water, (vi) dimethylformamide or dimethylsulfoxide, employed alone or as a mixture, spreading the said solution over an appropriate substrate and evaporating the solvent sₑ; and optionally crosslinking;
2) the preparation of at least on layer A according to the following process:
- preparation of a solution by mixing at least one active chemical substance x, optionally in a solvent sₑ, with a structuring polymer c, and optionally a bonding agent y,
- introduction of a crosslinking agent ri, directly in the solution,
- spreading the said solution over the layer obtained above in stage 1),
- initiation of the crosslinking reaction by the photochemical and/or thermal route or alternatively by contact with a crosslinking accelerator;
3) the spreading of a succession of layers A and E according to a desired order, which layers A and E are optionally separated by bonding layers Z, and are prepared in accordance with stages 1) and 2), and
4) the preparation and the spreading of a final elastomer barrier layer E, which may or may not carry a bonding agent y, by evaporating the solvent sₑ or the water, this stage optionally being followed by a crosslinking stage.

21. Process for the preparation of a multilayer material comprising at least one layer A composed of a non-reversible gel according to claim 1, **characterized in that** it comprises:
1) the preparation of a first elastomer barrier layer E, which may or may not carry a bonding agent y, by dissolution of a polymer e such as defined in claim 18, in a solvent sₑ as defined in claim 20, spreading the said solution over an appropriate substrate and evaporation of the solvent sₑ; and optionally crosslinking;
2) the preparation of at least on layer A according to the following process:
- preparation of a solution by mixing at least one active chemical substance x, optionally in a solvent sₑ, with a structuring polymer c, and optionally a bonding agent y,
- introduction, simultaneously with the mixture comprising the polymer c, the active chemical substance x, the solvent sₑ, and optionally the bonding agent y, of a crosslinking agent ri;
- spreading the said solution over the layer obtained stage 1), followed by rapid crosslinking, inducing the formation of the film;
3) the spreading of a succession of layers A and E according to a desired order, which layers A and E are optionally separated by bonding layers Z and are prepared in accordance with stages 1) and 2), and
4) the preparation and the spreading of a final elastomer barrier layer E, which may or may not carry a bonding agent y, by evaporating the solvent sₑ or the water, said stage being optionally followed by a crosslinking stage.

22. Preparation process according to claim 20 or 21, **characterized in that** the bonding layer Z is prepared by evaporation of the solvent s_{z} or of the water from a mixture comprising a polymer z, according to claim 19, in solution in a solvent s_{z}, selected from the group which comprises aromatic, aliphatic and alicyclic hydrocarbons, more polar compounds, such as ethers, such as tetrahydrofurane, dimethylformamide, dimethylsulfoxide or ketones, or alcohols, and water or a mixtures of intermediate polarity.

23. Preparation process according to claim 22, **characterized in that** the bonding layer Z is subjected to a crosslinking initiated photochemically and accelerated thermally or alternatively chemically.

24. Process for the preparation of a multilayer material comprising as layer A, a non-reversible gel according to claim 2, **characterized in that** it comprises a stage of preparation of the structuring polymer c by *in situ* polymerization from a mixtures of one or a number of monomers m and of at least one active chemical substance x, by the thermal, photochemical or radiochemical route.

25. Process according to claim 24, **characterized in that** the said mixture additionally comprises at least one of the following additional components: an initiator i, one or a number of solvents sₐ, a thickener f, a crosslinking agent ri, a bonding agent y.

26. Process according to claim 24, **characterized in that** the said monomers m are selected from the group consisting of acrylics, acrylates, acrylamides, acrylamines and their derivatives, vinylpyrrolidone.

27. Process according to claim 24, **characterized in that** the number of monomers used is between 1 and 10, preferably between 1 and 4.

28. Process according to claim 24 or claim 25, **characterized in that** in the case of a polymerization initiated thermally, the initiator i is selected from the group consisting of the family of peroxides, such as benzoyl peroxide, the family of azonitriles, such as azobisbutyronitrile, or redox pairs, such as the pair S₂O₈/S₂O₅.

29. Process according to claim 24 or claim 25, **characterized in that** in the case of a polymerization is initiated photochemically, the initiator i is selected from the group consisting of the family of acetophenones, benzoin derivatives or the family of peroxides.

30. Process according to claim 24 or claim 25, **characterized in that** the thickener f is selected from the group consisting of aluminium stearates, calcium linoleate pastes, hydrogenated castor oil, triglycerides, modified clays of bentonite type, polyol esters, silicas and polymers which are compatible with the mixtures of monomers or of the same nature as the gelled polymer.

31. Process for the preparation of a multilayer material according to claim 1, **characterized in that** it comprises stages of preparation of the layers A and E according to claims 20 to 30, a chemical or physical treatment of the said layers A and/or E being carried out after they have been spread over an appropriate substrate.

32. Process according to claim 31, **characterized in that** the said chemical treatment is carried out by grafting of by chemical attack with an acid solution and the physical treatment is carried out by bombardment of the surface of the film using ions, electrons or photons.

33. Applications of the multilayer material according to any one of claims 1 to 19 as coating for substrates, in particular made of elastomer or of plastic or for overmoulding a friction joint.

34. Glove, **characterized in that** it is coated with a multilayer material according to any one of claims 1 to 19.

35. Fingerstall, **characterized in that** it is coated with a multilayer material according to any one of claims 1 to 19.

36. Condom, **characterized in that** it is coated with a multilayer material according to any one of claims 1 to 19.

## Patentansprüche

1. Polymerer Mehrschichtwerkstoff, **dadurch gekennzeichnet, daß** er mindestens eine Schicht A aus einem Gel, das in Abhängigkeit von der Temperatur nicht-reversibel ist, und einen Zuschlag mindestens einer aktiven chemischen Substanz x aufweist, und mindestens zwei Sperrschichten E, welche ein synthetisches Elastomer e aufweisen, aufweist, wobei die Anbringung der Schichten aneinander durch einen in mindestens eine der Schichten A oder E eingebrachten polymeren Klebstoff y und/oder durch eine unabhängige Klebeschicht Z, welche ein Polymer z aufweist, und/oder eine chemische oder physikalische Behandlung mindestens einer der Schichten A oder E durchgeführt wird.

2. Mehrschichtwerkstoff nach Anspruch 1, **dadurch gekennzeichnet, daß** das nicht-reversible Gel im wesentlichen ein strukturbildendes Polymer c, ein Vernetzungsmittel ri, mindestens eine aktive chemische Substanz x, und ein Lösungsmittel sₐ aufweist.

3. Mehrschichtwerkstoff nach Anspruch 2, **dadurch gekennzeichnet, daß** das nicht-reversible Gel des weiteren einen Klebstoff y enthält.

4. Mehrschichtwerkstoff nach einem der Ansprüche 2 oder 3, **dadurch gekennzeichnet, daß** das strukturbildende Polymer c der Schicht A ein Polymer ist, das mit dem synthetischen Elastomer e nicht mischbar ist.

5. Mehrschichtwerkstoff nach einem der Ansprüche 2 bis 4, **dadurch gekennzeichnet, daß** das strukturbildende Polymer c der Schicht A vom hydrophilen Typ ist und mit der aktiven chemischen Substanz x, für sich oder in Lösung in einem Lösungsmittel sₐ, kompatibel ist.

6. Mehrschichtwerkstoff nach Anspruch 2 oder Anspruch 3, **dadurch gekennzeichnet, daß** das strukturbildende Polymer c der Schicht A ausgewählt ist aus der Gruppe bestehend aus (i) Polymeren natürlichen Ursprungs, die unter Gelatine, Gummis, Pektinen und Alginaten; Polypeptiden; Polyharnstoffen; Heparinoiden und Polygluconsäure, und Cellulosederivaten, die unter Methylcellulose, Hydroxyethylcellulose, Hydroxypropylcellulose und Carboxymethylcellulose ausgewählt sind; und (ii) synthetischen Polymeren, die unter Polyethylenimin, Polyacrylsäure, Polyvinylalkohol, für sich oder in einander durchdringenden Vernetzungen, Polyacrylamiden und deren Derivaten, Polyvinylpyrrolidon, Polydimethylsiloxan und Poly(vinylethern) ausgewählt sind.

7. Mehrschichtwerkstoff nach Anspruch 2, **dadurch gekennzeichnet, daß** das Vernetzungsmittel ri der Schicht A für Polymere c, die Hydroxylfunktionen aufweisen, unter den Aldehyden oder Dialdehyden mit einer niedrigen oder hohen relativen Molekülmasse und Harnstoffformaldehyd- oder Melaminformaldehydderivaten, und für Polymere, die Hydroxyl-, Carboxyl- oder Aminfunktionen aufweisen, unter Vorpolymeren, die Isocyanatfunktionen aufweisen, oder unter Methacrylaten, Imiden und Persulfaten ausgewählt ist.

8. Mehrschichtwerkstoff nach einem der Ansprüche 1 bis 7, **dadurch gekennzeichnet, daß** die in der Schicht A vorliegende aktive chemische Substanz x unter Verbindungen ausgewählt ist, die in der Lage sind, eine quasi augenblickliche Denaturierung von Proteinen durch einfachen Kontakt entweder aufqrund einer chemischen Reaktion oder eines physikalisch-chemischen Effektes wie etwa eine Modifikation der Oberflächenspannung hervorzurufen.

9. Mehrschichtwerkstoff nach Anspruch 8, **dadurch gekennzeichnet, daß** die chemische Substanz x unter den Biociden ausgewählt ist.

10. Mehrschichtwerkstoff nach Anspruch 9, **dadurch gekennzeichnet, daß** das Biocid aus der Gruppe bestehend aus quaternären Ammonia, Biguaniden, Phthalaldehyd, Phenolderivaten, Formaldehyd, nicht-ionischen Tensiden mit mindestens einer Polyoxyethylensequenz, Hexamidin, Iodverbindungen, nicht-ionischen Tensiden mit Virucidwirkung, Bichromaten und Hypochloriten von Natrium und Kalium oder mit einem Zuschlag versehenen Polymeren wie Polyelektrolyten mit Biocidwirkung wie Poly(acrylsäure), Divinylbenzyltrimethylammonium-Polychlorid, Divinylether-Maleinsäureanhydrid-Copolymer, Polyethyleniminen, Polyetheraminen, Polyharnstoffen und Polypeptiden ausgewählt ist, die entweder für sich oder in Mischung verwendet werden.

11. Mehrschichtwerkstoff nach Anspruch 10, **dadurch gekennzeichnet, daß** das Biocid Dimethyldidecylammonium ist.

12. Mehrschichtwerkstoff nach Anspruch 2, **dadurch gekennzeichnet, daß** das Lösungsmittel sₐ der Schicht A aus der Gruppe bestehend aus (i) schwach flüchtigen Verbindungen, die unter den Polyolen ausgewählt sind, (ii) Wasser, und (iii) flüchtigen Lösungsmitteln wie Alkoholen und Ketonen mit einer niedrigen relativen Molekülmasse, nicht-cyclischen oder cyclischen Etherverbindungen, Dimethylformamid, Dimethylsulfoxid ausgewählt ist, die entweder für sich oder in Mischung verwendet werden.

13. Mehrschichtwerkstoff nach Anspruch 1, **dadurch gekennzeichnet, daß** der Klebstoff y unter Polymeren ausgewählt ist, die gleichzeitig mindestens eine poly-A-Sequenz, die mit der Schicht A kompatibel ist, und mindestens eine poly-E-Sequenz, die mit der Schicht E kompatibel ist, aufweisen und aus der Gruppe bestehend aus Di-Blockcopolymeren vom Typ polyA-Block-polyE, Tri-Blockcopolymeren vom Typ polyE-Block-polyA-Block-polyE (EAE), vom Typ polyA-Block-polyE-Block-polyA (AEA), vom Typ polyA-Block-polyE-Block-polyF (AEF), vom Typ poly-A-Block-polyF-Block-polyE (AFE), und Pfropfcopolymeren vom Typ polyA-Pfropf-polyE, polyE-Pfropf-polyA oder vom Typ polyA-Pfropf-polyE und -polyF, wobei die -poly-F-Sequenz mit der Schicht A oder mit der Schicht E kompatibel ist, oder unter Klebstoffen vom Acryl-, Silicon- oder Polyurethantyp ausgewählt sind.

14. Mehrschichtwerkstoff nach Anspruch 13, **dadurch gekennzeichnet, daß** die poly-A-Sequenzen aus der Gruppe ausgewählt sind, die Polyoxyethylen, Polyvinylpyridin, Polyacrylsäuren, Polyvinylalkohol und quaternisiertes Polyvinylpyridin aufweist, und die poly-E-Sequenzen aus der Gruppe ausgewählt sind, die Polydiene, Polyolefine, Polyoxypropylen und Polydimethylsiloxan aufweist.

15. Mehrschichtwerkstoff nach einem der Ansprüche 1 bis 7, **dadurch gekennzeichnet, daß** die Schicht A aus Gel aufweist:
- zwischen 0 und 98% Lösungsmittel sₐ bezogen auf das Gel
- zwischen 0,1 und 74% strukturbildendes Polymer c bezogen auf das Gel
- zwischen 1 und 80% der aktiven chemischen Substanz x bezogen auf das Gel, und
- zwischen 0,0001 und 0,1% Vernetzungsmittel ri (Masseanteil Vernetzungsmittel/Inhaltsstoffe insgesamt).

16. Mehrschichtwerkstoff nach Anspruch 15, **dadurch gekennzeichnet, daß** die Schicht A aus Gel aufweist:
- zwischen 25 und 98% Lösungsmittel sₐ bezogen auf das Gel
- zwischen 1 und 74% strukturbildendes Polymer c bezogen auf das Gel
- zwischen 1 und 74% der aktiven chemischen Substanz x bezogen auf das Gel.

17. Mehrschichtwerkstoff nach Anspruch 15, **dadurch gekennzeichnet, daß**, wenn die Schicht A aus Gel des weiteren einen Klebstoff y aufweist, dessen Masseanteile zwischen 0,01 und 25% bezogen auf das Gel liegen, wobei die Anteile von Lösungsmittel sₐ entsprechend angepaßt sind.

18. Mehrschichtwerkstoff nach einem der Ansprüche 1 bis 17, **dadurch gekennzeichnet, daß** die Elastomerschichten E ein synthetisches Elastomer e, das unter Polybutadien, Polyisopren, Acrylpolymeren, Polychloropren, Polyurethan, Copolymeren auf Basis von Chlorbutadien und Methacrylsäure oder auf Basis von Ethylen und Vinylacetat, SBR (*Styrene Butadiene Rubber*)-, SBS (*Styrene Butadiene Styrene*)-, SIS (*Styrene Isoprene Styrene*)- oder SEBS *(Styrene Ethylene Butylene Styrene*)-Copolymeren ausgewählt ist, und gegebenenfalls einen Klebstoff y gemäß der Definition in Anspruch 13 oder Anspruch 14 aufweisen.

19. Mehrschichtwerkstoff nach Anspruch 1, **dadurch gekennzeichnet, daß** das Polymer z der Klebeschicht Z ausgewählt ist aus der Gruppe bestehend aus (i) Di-Block-, Tri-Block- oder Pfropfcopolymeren nach Anspruch 13, (ii) Polymeren vom Acryl- oder Vinyltyp, (iii) Dienpolymeren mit Nitrilfunktionen wie NBR (*Nitrile Butadiene Rubber*), Polyurethanen und bestimmten Polyestern oder Polyamiden, und (iv) Copolymeren vom gleichen Typ wie die für die Schicht E gewählten, welche, um sie mit der Schicht A kompatibel zu machen, entweder chemisch durch Pfropfen oder chemischen Angriff mit einer Säurelösung oder auch physikalisch durch Beschuß der Oberfläche des Films mittels Ionen, Elektronen oder Photonen behandelt sind.

20. Verfahren zur Herstellung eines Mehrschichtwerkstoffes mit mindestens einer Schicht A aus einem nichtreversiblen Gel nach Anspruch 1, **dadurch gekennzeichnet, daß** es aufweist:
1) Herstellen einer ersten Sperrschicht aus Elastomer E, die einen Zuschlag eines Klebstoffs y aufweisen kann, durch Auflösen eines Polymers e gemäß der Definition in Anspruch 18 in einem Lösungsmittel sₑ, ausgewählt aus der Gruppe bestehend aus (i) aromatischen, aliphatischen und alicyclischen Kohlenwasserstoffen, beispielsweise Paraffinkohlenwasserstoffen, Cyclohexan, Benzol, Toluol, Xylol, Tetralin, Decalin, (ii) Erdölfraktionen, (iii) weiteren Lösungsmitteln mit einem höheren Polaritätsgrad wie Alkoholen oder Ketonen mit einer niedrigen relativen Molekülmasse, (iv) nichtcyclischen oder cyclischen Etherverbindungen, (v) Wasser, (vi) Dimethylformamid oder Dimethylsulfoxid, die entweder für sich oder in Mischungen verwendet werden, Ausbreiten der Lösung auf einem geeigneten Träger, und Verdampfen des Lösungsmittels sₑ; und gegebenenfalls Vernetzung;
2) Herstellen mindestens einer Schicht A gemäß dem nachfolgenden Verfahren:
- Herstellen einer Lösung durch Mischen von mindestens einer aktiven chemischen Substanz x, gegebenenfalls in einem Lösungsmittel sₐ, mit einem strukturbildenden Polymer c und gegebenenfalls einem Klebstoff y,
- Einbringen eines Vernetzungsmittels ri unmittelbar in die Lösung,
- Ausbreiten der Lösung auf der in Schritt 1) erhaltenen Schicht,
- Initiieren der Vernetzung auf photochemischem und/oder thermischem Wege oder durch Kontakt mit einem Vernetzungsbeschleuniger;
3) Ausbreiten einer Abfolge von Schichten A und E gemäß einer gewünschten Reihenfolge, die durch Klebeschichten Z getrennt sein können oder auch nicht, wobei die Schichten A und E gemäß den Schritten 1) und 2) hergestellt sind, und
4) Herstellen und Ausbreiten einer abschließenden Sperrschicht aus Elastomer E, die einen Zuschlag aus einem Klebstoff y aufweisen kann, durch Verdampfen des Lösungsmittels sₑ oder Wassers, wobei dieser Schritt gegebenenfalls von einem Vernetzungsschritt gefolgt ist.

21. Verfahren zur Herstellung eines Mehrschichtwerkstoffes mit mindestens einer Schicht A aus einem nichtreversiblen Gel nach Anspruch 1, **dadurch gekennzeichnet, daß** es aufweist:
1) Herstellen einer ersten Sperrschicht aus Elastomer E, die einen Zuschlag eines Klebstoffs y aufweisen kann, durch Auflösen eines Polymers e gemäß der Definition in Anspruch 18 in einem Lösungsmittel sₑ gemäß der Definition in Anspruch 20, Ausbreiten der Lösung auf einem geeigneten Träger und Verdampfen des Lösungsmittel sₑ; und gegebenenfalls Vernetzung;
2) Herstellen mindestens einer Schicht A gemäß dem nachfolgenden Verfahren:
- Herstellen einer Lösung durch Mischen mindestens einer aktiven chemischen Substanz x, gegebenenfalls in einem Lösungsmittel sₐ, mit einem strukturbildenden Polymer c und gegebenenfalls einem Klebstoff y,
- gleichzeitiges Einbringen eines Vernetzungsmittels ri in die Mischung, welche das strukturbildende Polymer c, die aktive chemische Substanz x, das Lösungsmittel sₐ und gegebenenfalls den Klebstoff y aufweist,
- Ausbreiten der Lösung auf der in Schritt 1) erhaltenen Schicht, gefolgt von einer schnellen Vernetzung, welche die Bildung des Films induziert;
3) Ausbreiten einer Abfolge von Schichten A und E gemäß einer gewünschten Reihenfolge, die durch Klebeschichten Z getrennt sein können oder auch nicht, wobei die Schichten A und E gemäß den Schritten 1) und 2) hergestellt sind, und
4) Herstellen und Ausbreiten einer abschließenden Sperrschicht aus Elastomer E, die einen Zuschlag eines Klebstoffs y aufweisen kann, durch Verdampfen des Lösungsmittels sₑ oder des Wassers, wobei dieser Schritt gegebenenfalls von einem Vernetzungsschritt gefolgt ist.

22. Herstellungsverfahren nach Anspruch 20 oder 21, **dadurch gekennzeichnet, daß** die Klebeschicht Z hergestellt wird durch Abdampfen des Lösungsmittels s_{z} oder des Wassers aus einer Mischung, welche ein Polymer z nach Anspruch 19 aufweist, in Lösung in einem Lösungsmittel s_{z}, das aus der Gruppe ausgewählt ist, welche aromatische, aliphatische und alicyclische Kohlenwasserstoffe, Verbindungen mit einem höheren Polaritätsgrad wie etwa Ether wie Tetrahydrofuran, Dimethylformamid, Dimethylsulfoxid oder Ketone, Alkohole, sowie Wasser oder eine Mischung mit einem mittleren Polaritätsgrad aufweist.

23. Herstellungsverfahren nach Anspruch 22, **dadurch gekennzeichnet, daß** die Klebeschicht Z eine photochemisch initiierte und thermisch oder chemisch beschleunigte Vernetzung subit.

24. Verfahren zur Herstellung eines Mehrschichtwerkstoffes, welcher als Schicht A ein nicht-reversibles Gel nach Anspruch 2 enthält, **dadurch gekennzeichnet, daß** es einen Schritt zur Herstellung des strukturbildenden Polymers c durch Polymerisation *in situ* ausgehend von einer Mischung von einem oder mehreren Monomeren m und mindestens einer aktiven chemischen Substanz x auf thermischem, photochemischem oder radiochemischem Wege aufweist.

25. Verfahren nach Anspruch 24, **dadurch gekennzeichnet, daß** die Mischung des weiteren mindestens einen der folgenden zusätzlichen Inhaltsstoffe aufweist: ein Initiierungsmittel i, ein oder mehrere Lösungsmittel sₐ, ein Eindickungsmittel f, ein Vernetzungsmittel ri, einen Klebstoff y.

26. Verfahren nach Anspruch 24, **dadurch gekennzeichnet, daß** die Monomere m in der Gruppe bestehend aus Acrylen, Acrylaten, Acrylamiden, Acrylaminen und deren Derivaten, Vinylpyrrolidon ausgewählt sind.

27. Verfahren nach Anspruch 24, **dadurch gekennzeichnet, daß** die Anzahl der verwendeten Monomere zwischen 1 und 10, bevorzugt zwischen 1 und 4 liegt.

28. Verfahren nach Anspruch 24 oder Anspruch 25, **dadurch gekennzeichnet, daß** im Falle einer thermisch initiierten Polymerisation das Initiierungsmittel i aus der Gruppe bestehend aus der Familie der Peroxide wie Benzoylperoxid, der Familie der Azonitrile wie Azobisbutyronitril, oder Redoxpaaren wie dem Paar S₂O₈/S₂O₅ ausgewählt ist.

29. Verfahren nach Anspruch 24 oder Anspruch 25, **dadurch gekennzeichnet, daß** im Fall einer auf photochemischem Wege initiierten Polymerisation das Initiierungsmittel i aus der Gruppe bestehend aus der Familie der Acetophenone, den Derivaten von Benzoin, oder der Familie der Peroxide ausgewählt ist.

30. Verfahren nach Anspruch 24 oder Anspruch 25, **dadurch gekennzeichnet, daß** das Eindickungsmittel f aus der Gruppe ausgewählt ist bestehend aus Aluminiumstearaten, Pasten von Calciumlinoleat, hydrogeniertem Ricinusöl, Triglyceriden, modifizierten Tonen vom Typ Bentonit, Polyolestern, Silicamassen und Polymeren, die kompatibel mit der Mischung von Monomeren oder von der gleichen Art wie das gelierte Polymer sind.

31. Verfahren zur Herstellung eines Mehrschichtwerkstoffs nach Anspruch 1, **dadurch gekennzeichnet, daß** es Schritte zur Herstellung der Schichten A und E gemäß den Ansprüchen 20 bis 30 aufweist, wobei eine chemische oder physikalische Behandlung der Schichten A und/oder E nach ihrem Ausbreiten auf einem geeigneten Träger durchgeführt wird.

32. Verfahren nach Anspruch 31, **dadurch gekennzeichnet, daß** die chemische Behandlung mittels Pfropfen oder durch chemischen Angriff mit einer Säurelösung durchgeführt wird, und die physikalische Behandlung mittels Beschuß der Oberfläche des Films mittels Ionen, Elektronen oder Photonen durchgeführt wird.

33. Anwendungen des Mehrschichtwerkstoffs nach einem der Ansprüche 1 bis 19 als Beschichtung von Trägern insbesondere aus Elastomer oder Plastik, oder Aufformung einer Abstreifdichtung.

34. Handschuh, **dadurch gekennzeichnet, daß** er mit einem Mehrschichtwerkstoff nach einem der Ansprüche 1 bis 19 beschichtet ist.

35. Fingerling, **dadurch gekennzeichnet, daß** es mit einem Mehrschichtwerkstoff nach einem der Ansprüche 1 bis 19 beschichtet ist.

36. Präservativ, **dadurch gekennzeichnet, daß** es mit einem Mehrschichtwerkstoff nach einem der Ansprüche 1 bis 19 beschichtet ist.
